# EUROPEAN PATENT SPECIFICATION

(11) **EP 2 119 766 B1**
(45) Date of publication and mention of the grant of the patent: **15.06.2016**
(21) Application number: 08711097.9
(22) Date of filing: 12.02.2008
(51) Int. Cl.: C12N 1/20, C12N 1/04, A23C 9/13, A23L 3/358

(54) **AGENT FOR IMPROVING VIABILITY OF LACTIC ACID BACTERIUM**
MITTEL FÜR ERHÖHTE LEBENSFÄHIGKEIT VON MILCHSÄUREBAKTERIEN
PROCÉDÉ POUR AMÉLIORER LA VIABILITÉ D'UNE BACTÉRIE D'ACIDE LACTIQUE

(30) Priority: 16.02.2007 JP 2007036277
(43) Date of publication of application: 18.11.2009
(73) Proprietor: MEGMILK SNOW BRAND Co., Ltd., Higashi-ku Sapporo (JP)
(72) Inventor: MIURA, Mari, Kawagoe-shi Saitama 350-1165 (JP); SETO, Yasuyuki, Kawagoe-shi Saitama 350-1165 (JP); WATANABE, Masayuki, Kawagoe-shi Saitama 350-1165 (JP); YOSHIOKA, Toshimitsu, Kawagoe-shi Saitama 350-1165 (JP)
(74) Representative: Andrae | Westendorp Patentanwälte Partnerschaft
(86) International application number: PCT/JP2008/052233
(87) International publication number: WO 2008/099801

(56) References cited:
- EP-A1- 0 649 603
- JP-A- 11 004 665
- RACCACH M. ET AL.: 'Manganese and lactic acid bacteria' J. FOOD PROT. vol. 48, no. 10, 1985, pages 895 - 898, XP008110593
- A.S. Carvalho ET AL: "Effect of various growth media upon survival during storage of freeze-dried Enterococcus faecalis and Enterococcus durans", Journal of Applied Microbiology, vol. 94, no. 6, 1 June 2003 (2003-06-01), pages 947-952, XP055101123, ISSN: 1364-5072, DOI: 10.1046/j.1365-2672.2003.01853.x

## Description

### Technical field

The present invention relates to the use of manganese for improving the survival rate upon storage of lactic acid bacteria, as well as to the use of a manganese-containing material for improving the survival rate upon storage of lactic acid bacteria. Furthermore, the present invention pertains to the use of manganese or manganese-containing material for improving the viability of lactic acid bacteria in a product subjected to storage, the product being selected from the group consisting of a fermented milk, a dairy products lactic acid bacteria drink, and a lactic acid bacteria drink.

### Background Art

Various physiological effects of lactic acid bacteria, such as intestinal regulation effect, defense against infection, immunostimulatory action and cancer prevention, have been found one after another, and development for using lactic acid bacteria or cultures thereof as materials for health foods, drugs or the like has been made. Moreover, recent studies have found that the above-mentioned functionalities are improved by delivering the lactic acid bacteria in a viable condition to the intestines, and the improvement of the viability in the product also has great industrial advantages in applications to Foods for Specified Health Use rapidly increasing the demand in recent years and the like.

However, the viability of lactic acid bacteria has been affected by the bacterial strain, culture phase, product pH, the concentration of sugar used as a sweetener, or the like, and it has been very difficult to improve the viability of lactic acid bacteria while keeping the original taste of the product. Therefore, a special container that suppresses oxygen permeability as much as possible or the like has been used, but the special container has a problem of increase in the cost. Moreover, as a method of producing a low-fat yogurt causing little decrease in lactic acid bacteria during storage, a method of improving the survival rate of lactic acid bacteria by adding oleic acid, or a salt or ester thereof to a fermentation base has been disclosed (for example, see Patent Document 1). However, most of oleic acid-related compounds that can be used as food additives in the country have distinctive smells, and the use of the compounds inevitably results in the deterioration of the flavor of the products, and the satisfactory survival rate have not been obtained.

Furthermore, a method of producing a liquid yogurt with low viscosity and high viability by adding peroxidase to a raw material mixture has been disclosed (for example, see Patent Document 2), but the addition of the above material affects the cost of the product.
Patent Document 1: Japanese Patent Laid-Open No. 2001-45968
Patent Document 2: Japanese Patent Laid-Open No. 10-262550

EP 0 649 603 A1 discloses an antioxidant food comprising a product of fermentation of a food containing a manganese-containing natural material by using a microbe having a catalase activity. It has both a superoxide dismutase-like activity and a catalase activity and hence EP 0 649 603 A1 assumes that it exhibits an antioxidant activity in the body of a living organism, including the digestive tract. Therefore EP 0 649 603 A1 assumes that it is efficacious in preventing diseases caused by active oxygen.

M. Raccach describes in the publication entitled "Manganese and Lactic Acid Bacteria" (Journal of Food Protection, Vol. 48, No. 10, pages 895 to 898 (1985)) that Mn (manganese) accelerates growth and acid production by LAB (lactic acid bacteria).

A. S. Carvalho et al. discuss the effect of various growth media upon survival during storage of freeze-dried Enterococcus faecalis and Enterococcus durans (Journal of Applied Microbiology, vol. 94, no. 6, 2003-06-01, pages 947-952).

### Disclosure of the Invention

### Problems to be Solved by the Invention

A substance having the effect of improving the viability of lactic acid bacteria which is obtained using a conventional technology, has problems that, since the substance itself is expensive or has a peculiar taste or odor, only a very small amount of the substance can be added to a medium in producing the products. Therefore, in consideration of the above problems, an object of the present invention is to provide an agent which can be used for improving the viability of lactic acid bacteria, which has an excellent effect on the improvement of the viability of lactic acid bacteria in the product, has no adverse effect on the flavor of the product and requires less production cost.

### Means for Solving the Problems

The inventors of the present invention have made extensive studies in order to obtain an agent for improving the viability of lactic acid bacteria, which has an excellent effect on the improvement of the viability of lactic acid bacteria and has no adverse effect on the flavor of the product and the cost. As a result, the inventors have found that manganese has the effect of markedly improving the viability of lactic acid bacteria.

More specifically, the inventors have found that the use of manganese maintains the high number of viable lactic acid bacteria to be grown in the product without impairing the flavor of the product and while preventing increase in the cost due to the use of a special and expensive container that suppresses oxygen permeability as much as possible or the like, thereby completing the present invention.

Disclosed herein is:
(1) an agent for improving the viability of lactic acid bacteria using manganese as an effective ingredient;
(2) an agent for improving the viability of lactic acid bacteria using a manganese-containing material as an effective ingredient; and
(3) a fermented milk, a dairy products lactic acid bacteria drink or a lactic acid bacteria drink, wherein manganese is formulated at 5 µg/100 g or more in the final product.

According to an aspect, the present invention provides the use of manganese for improving the survival rate upon storage of lactic acid bacteria, as defined in claim 1 annexed.

According to a further aspect, the present invention provides the use of a manganese-containing material for improving the survival rate upon storage of lactic acid bacteria, as defined in claim 2 annexed.

According to a further aspect, the present invention provides the use of manganese for improving the viability of lactic acid bacteria in a product subjected to storage, as defined in claim 7 annexed.

According to a further aspect, the present invention provides the use of manganese-containing material for improving the viability of lactic acid bacteria in a product subjected to storage, as defined in claim 9 annexed.

### Advantage of the Invention

By adding manganese, the high number of viable lactic acid bacteria can be maintained in the product without impairing the flavor of the product and while preventing increase in the cost due to the use of a special and expensive container that suppresses oxygen permeability as much as possible or the like.

### Brief Description of the Drawings

Figure 1 shows the effect of manganese on the viability of Lactobacillus casei, Lactobacillus helveticus, and Pediococcus pentosaceus (Example 1);
Figure 2 shows the effect of the amount of manganese on the viability of Lactobacillus casei (Example 2);
Figure 3 shows the effect of the amount of manganese on the survival rate of Lactobacillus casei (day 21 of storage) (Example 2); and
Figure 4 shows the effect of the addition of manganese yeast on the viability of Lactobacillus casei (Example 3).

### Best Mode for Carrying Out the Invention

As the manganese in the present invention, besides manganese sulfate, manganese yeast, manganese lactic acid bacteria and the like can be used. As the manganese-containing material in the present invention, manganese-containing materials such as tea leaves such as black tea, broiled green tea, refined green tea and the like, ginger, green laver, and acidic buttermilk can be used.

The agent for improving the viability of lactic acid bacteria can be used as an
aqueous solution as is or used in the state of a concentrated solution or powder. The acidic buttermilk obtained in the production of fermented butter produced by fermentation using lactic acid bacteria can be also used as an aqueous solution as is or as a concentrated solution or powder. In addition, the tea leaves, ginger and the like can be also used as a powder or used as an extract.

The agent for improving the viability of lactic acid bacteria is characterized by
using the manganese or the manganese-containing material as an effective ingredient, and other foods, food materials or known food additives can be also used as required.

The lactic acid bacteria in the present invention are not particularly limited, and any lactic acid bacteria can be used as long as those widely used for conventionally known fermented foods, fermented milk or the like. As the above-mentioned lactic acid bacteria, any lactic acid bacteria of Lactobacilli such as the genera of Lactobacillus and Weissella, and Lactococci such as the genera of Pediococcus, Leuconostoc, Lactococcus, Streptococcus, and Enterococcus is effective, and plural lactic acid bacteria can be used in combination.

The agent for improving the viability of lactic acid bacteria can be directly added as is to a fermentation mixture for a fermented milk product and can be also used by adding to a seed or bulk starter of the lactic acid bacteria to be grown. In addition, the agent for improving the viability of lactic acid bacteria achieves an effect by being added to the lactic acid bacteria to be grown before fermentation or after fermentation.

As the amount of manganese that achieves an effect as the agent for improving the viability of lactic acid bacteria, it is characterized in that the manganese is added in an amount to be 5 µg/100 g or more, preferably 10 µg/100 g or more, and more preferably 100 µg/100 g or more in the final product. An amount less than 5 µg/100 g does not achieve the full effect of improving the viability of lactic acid bacteria.

A yogurt drink is prepared by mixing a lactic acid bacteria fermented product with sugar syrup or the like. Therefore, manganese is contained at 5 µg/100 g or more in the final product, whereby the effect of improving the viability can be achieved. Incidentally, the amount of manganese was measured using an inductively-coupled plasma (ICP) atomic emission spectrometer.

When manganese is added, the acid production rate is markedly increased as compared with a normal skim milk medium or the like. Therefore, the fermentation time can be also shortened.

The final product includes
a fermented milk, a dairy products lactic acid bacteria drink or a lactic acid bacteria drink but is not particularly limited thereto. The present invention can be used for foods and drinks using lactic acid bacteria.

As described above, the viability of lactic acid bacteria in the product can be easily improved by adding the manganese in the present invention, and it is not necessary to use a special container that suppresses oxygen permeability as much as possible and maintain pH around neutral. Therefore, the product can be produced in a short time without impairing the original flavor of the product while preventing increase in cost.

### Examples

Hereinafter, the present invention will be described in detail by showing Examples. The examples described below are intended to describe the present invention, and the present invention is not limited to the description of the examples.

### (Example 1)

The amount 1,600 g of skim milk (containing 0.02 mg/100 g manganese), 700 g of isomerized sugar (manufactured by Oji Cornstarch Co., Ltd.), 3 g of manganese sulfate and water were mixed so as to have a weight of 10 kg, and the mixture was sterilized at 95°C for 90 minutes to prepare a fermentation mixture.

Any of Lactobacillus casei ATCC 334, Lactobacillus helveticus JCM 1120 and Pediococcus pentosaceus JCM 5890 was inoculated into each fermentation mixture at a concentration of 0.5% by weight. Pediococcus pentosaceus JCM 5890 was fermented at 30°C until the acidity reached 1.0%, and other bacterial strains were fermented at 37°C until the acidity reached 2.1%. After the termination of fermentation, 10 kg of the culture was mixed with 40 kg of isomerized sugar (manufactured by Oji Cornstarch Co., Ltd.) to prepare a yogurt drink with BRIX of 15%. The resulting product was defined as a disclosed product. The yogurt drink was stored at 15°C, and the number of the lactic acid bacteria was counted on days 0, 7, 14, and 21. In the same manner, a yogurt drink was prepared without adding manganese sulfate and defined as a control product. Incidentally, the amount of manganese in the final product was 0.64 µg/100 g in the control products and 2.2 mg/100 g in the disclosed products.

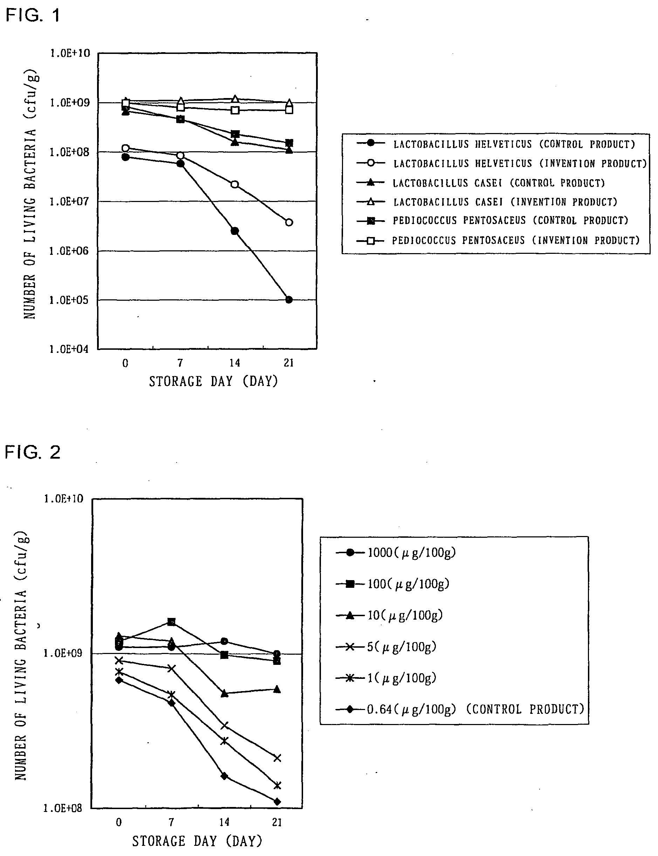

The effect of the addition of manganese on the viability of Lactobacillus casei ATCC 334, Lactobacillus helveticus JCM 1120 and Pediococcus pentosaceus JCM 5890 is shown in Figure 1. As a result, the viability was markedly improved in the invention products to which manganese was added as compared with the control products. Also, no effect on the flavor of the product was found.

### (Example 2)

The amount 0.49, 5.99, 12.8, 136.4 and 1372 mg of manganese sulfate were added respectively to 1,600 g of skim milk (containing 0.02 mg manganese/100 g) and 700 g of isomerized sugar (manufactured by Oji Cornstarch Co., Ltd.), and the mixture was mixed with water so as to have a weight of 10 kg and sterilized at 95°C for 90 minutes to prepare each fermentation mixture.

Lactobacillus casei ATCC 334 was inoculated into each fermentation mixture at a concentration of 0.5% by weight and thereafter fermented at 37°C until the acidity reached 2.1%. After the termination of fermentation, 10 kg of the culture was mixed with 40 kg of isomerized sugar (manufactured by Oji Cornstarch Co., Ltd.) to prepare a yogurt drink with BRIX of 15%. The yogurt drink was stored at 15°C, and the number of the lactic acid bacteria was counted on days 0, 7, 14, and 21. In the same manner, a yogurt drink was prepared without adding manganese sulfate and defined as a control product. Incidentally, the amount of manganese in the final product was 0.64 µg/100 g in the control product and 1, 5, 10, 100 and 1000 µg/100 g in the prepared products.

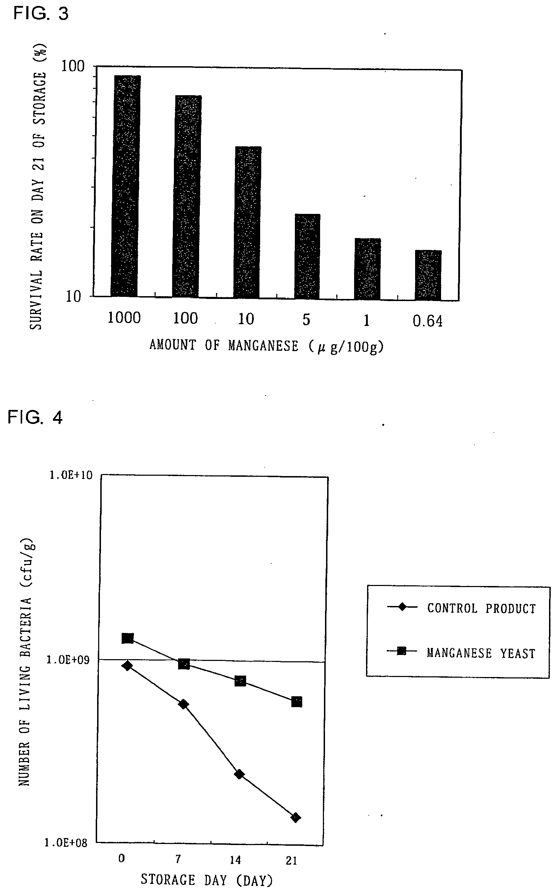

The effect of the amount of manganese on the viability of Lactobacillus casei ATCC 334 is shown in Figure 2, and the survival rate on day 21 of storage is shown in Figure 3. As a result, the more effect of adding manganese was found in the prepared product containing 5 µg manganese/100 g as compared with the control product. In the prepared product containing 10 µg manganese/100 g or more, the viability was markedly improved and the survival rate on day 21 of storage was 2.8 times as the control. Also, no effect on the flavor of the product was found.

### (Example 3)

The amount 1,600 g of skim milk (containing 0.02 mg manganese/100 g), 700 g of isomerized sugar (manufactured by Oji Cornstarch Co., Ltd.), 5 g of manganese yeast (manufactured by LALMIN, MN50) and water were mixed so as to have a weight of 10 kg, and the mixture was sterilized at 95°C for 90 minutes to prepare a fermentation mixture.

Lactobacillus casei ATCC 334 was inoculated into each fermentation mixture at a concentration of 0.5% by weight and thereafter fermented at 37°C until the acidity reached 2.1%. After the termination of fermentation, 10 kg of the culture was mixed with 40 kg of isomerized sugar (manufactured by Oji Cornstarch Co., Ltd.) to prepare a yogurt drink with BRIX of 15%. The resulting product was defined as an invention product. The yogurt drink was stored at 15°C, and the number of the lactic acid bacteria was counted on days 0, 7, 14, and 21. In the same manner, a yogurt drink was prepared without adding manganese sulfate and defined as a control product. Incidentally, the amount of manganese in the final product was 0.64 µg/100 g in the control product and 0.5 mg/100 g in the invention product.

The effect of the addition of the manganese yeast on the viability of Lactobacillus casei ATCC 334 is shown in Figure 4. As a result, the viability was markedly improved in the invention product to which the manganese yeast was added as compared with the control product. Also, no effect on the flavor of the product was found.

### (Example 4)

The amount 1,600 g of skim milk (containing 0.02 mg manganese/100 g), 700 g of isomerized sugar (manufactured by Oji Cornstarch Co., Ltd.) and 7,700 g of water were mixed, and the mixture was sterilized at 95°C for 90 minutes to prepare a fermentation mixture.

Lactobacillus helveticus JCM 1120 was inoculated into each fermentation mixture at a concentration of 3% and thereafter fermented at 37°C until the acidity reached 2.1%. The amount 10 kg of the culture was mixed with 40 kg of isomerized sugar (manufactured by Oji Cornstarch Co., Ltd.), and 500 mg of manganese sulfate was further added thereto to prepare a yogurt drink with BRIX of 15%. The resulting product was defined as an invention product. In the same manner, a yogurt drink was prepared without adding manganese sulfate and defined as a control product. Incidentally, the amount of manganese in the final product was 0.64 µg/100 g in the control product and 36 µg/100 g in the invention product. The yogurt drinks were stored at 10°C, and the number of the lactic acid bacteria was counted on days 0, 7, 14, and 21.

As a result, the survival rate was improved about 30 times in the invention product to which the manganese was added as compared with the control product. Also, no effect on the flavor of the product was found.

### (Example 5)

The amount 2,000 g of raw milk (containing 0.002 mg manganese/100 g), 800 g of skim milk (containing 0.02 mg manganese/100 g), 1,000 g of sugar, 30 mg of manganese sulfate and water were mixed so as to have a weight of 10 kg, and the mixture was sterilized at 95°C for 20 minutes to prepare a fermentation mixture.

Lactobacillus helveticus JCM 1120 was inoculated into each fermentation mixture at a concentration of 3% and thereafter fermented at 37°C until the acidity reached 0.8% to prepare a fermented milk. The resulting product was defined as an invention product. The fermented milk was stored at 10°C, and the number of the lactic acid bacteria was counted on days 0, 7, 14, and 21. In the same manner, a fermented milk was prepared without adding manganese sulfate and defined as a control product. Incidentally, the amount of manganese in the final product was 2 µg/100 g in the control product and 109 µg/100 g in the invention product.

As a result, the survival rate was improved about 10 times in the invention product to which the manganese was added as compared with the control product. Also, no effect on the flavor of the product was found.

### Industrial Applicability

The agent for improving the viability of lactic acid bacteria has an excellent effect on the improvement of the viability of lactic acid bacteria in the product, has no adverse effect on the flavor of the product and requires less production cost, so that the agent is very useful.

## Claims

1. Use of manganese for improving the survival rate upon storage of lactic acid bacteria,
wherein the lactic acid bacteria are lactic acid bacteria selected from the group consisting of lactic acid bacteria of the genus Lactobacillus and lactic acid bacteria of the genus Pediococcus, and
wherein the manganese is added in an amount to be from 10 µg/100g to 1000 µg/100g in a final product, the final product being selected from the group consisting of a fermented milk, a dairy products lactic acid bacteria drink, and a lactic acid bacteria drink.

2. Use of a manganese-containing material for improving the survival rate upon storage of lactic acid bacteria,
wherein the lactic acid bacteria are lactic acid bacteria selected from the group consisting of lactic acid bacteria of the genus Lactobacillus and lactic acid bacteria of the genus Pediococcus, and
wherein the manganese is added in an amount to be from 10 µg/100g to 1000 µg/100g in a final product, the final product being selected from the group consisting of a fermented milk, a dairy products lactic acid bacteria drink, and a lactic acid bacteria drink.

3. Use according to any of claims 1 to 2, wherein the use is for improving the survival rate upon storage of lactic acid bacteria in a yogurt drink.

4. Use according to any of claims 1 to 3, wherein said lactic acid bacteria are selected from the group consisting of Lactobacillus casei, Lactobacillus helveticus, and Pediococcus.

5. Use according to any of claims 1, 3 to 4, wherein as manganese one of manganese sulfate, manganese yeast and manganese lactic acid bacteria is used.

6. Use according to any of claims 2 to 4, wherein said manganese-containing material is selected from the group consisting of tea leaves, ginger, green laver and acidic buttermilk.

7. Use of manganese for improving the viability of lactic acid bacteria in a product subjected to storage, the product being selected from the group consisting of a fermented milk, a dairy products lactic acid bacteria drink, and a lactic acid bacteria drink, wherein manganese is formulated at from 10 µg/100g to 1000 µg/100g in the product selected from the group consisting of a fermented milk, a dairy products lactic acid bacteria drink, and a lactic acid bacteria drink, and
wherein the lactic acid bacteria are lactic acid bacteria selected from the group consisting of lactic acid bacteria of the genus Lactobacillus and lactic acid bacteria of the genus Pediococcus.

8. Use according to claim 7, wherein the product subjected to storage is a yogurt drink subjected to storage or a fermented milk subjected to storage.

9. Use of manganese-containing material for improving the viability of lactic acid bacteria in a product subjected to storage, the product being selected from the group consisting of a fermented milk, a dairy products lactic acid bacteria drink, and a lactic acid bacteria drink, wherein manganese is formulated at from 10 µg/100g to 1000 µg/100g in the product selected from the group consisting of a fermented milk, a dairy products lactic acid bacteria drink, and a lactic acid bacteria drink, and
wherein the lactic acid bacteria are lactic acid bacteria selected from the group consisting of lactic acid bacteria of the genus Lactobacillus and lactic acid bacteria of the genus Pediococcus.

10. Use according to claim 9, wherein the product subjected to storage is a yogurt drink subjected to storage or a fermented milk subjected to storage.

## Patentansprüche

1. Verwendung von Mangan zum Verbessern der Überlebensrate bei Lagerung von Milchsäurebakterien,
wobei die Milchsäurebakterien Milchsäurebakterien sind, die aus der Gruppe ausgewählt sind, welche aus Milchsäurebakterien der Gattung Lactobacillus und Milchsäurebakterien der Gattung Pediococcus besteht, und
wobei das Mangan in einer Menge zugegeben wird, so dass es im Endprodukt 10 µg/100 g bis 1000 µg/100 g umfasst, wobei das Endprodukt aus der Gruppe ausgewählt ist, die aus einer fermentierten Milch, einem Molkereiprodukt-Milchsäurebakteriengetränk und einem Milchsäurebakteriengetränk besteht.

2. Verwendung eines manganhaltigen Materials zum Verbessern der Überlebensrate bei Lagerung von Milchsäurebakterien,
wobei die Milchsäurebakterien Milchsäurebakterien sind, die aus der Gruppe ausgewählt sind, welche aus Milchsäurebakterien der Gattung Lactobacillus und Milchsäurebakterien der Gattung Pediococcus besteht, und
wobei das Mangan in einer Menge zugegeben wird, so dass es im Endprodukt 10 µg/100 g bis 1000 µg/100 g umfasst, wobei das Endprodukt aus der Gruppe ausgewählt ist, die aus einer fermentierten Milch, einem Molkereiprodukt-Milchsäurebakteriengetränk und einem Milchsäurebakteriengetränk besteht.

3. Verwendung gemäß einem der Ansprüche 1 bis 2, wobei die Verwendung zum Verbessern der Überlebensrate bei Lagerung von Milchsäurebakterien in einem Yoghurtgetränk ist.

4. Verwendung gemäß einem der Ansprüche 1 bis 3, wobei die Milchsäurebakterien aus der Gruppe ausgewählt sind, welche aus Lactobacillus casei, Lactobacillus helveticus und Pediococcus besteht.

5. Verwendung gemäß einem der Ansprüche 1, 3 bis 4, wobei als Mangan eines von Mangansulfat, Manganhefe und Manganmilchsäurebakterien verwendet wird.

6. Verwendung gemäß einem der Ansprüche 2 bis 4, wobei das manganhaltige Material aus der Gruppe ausgewählt ist, welche aus Teeblättern, Ingwer, Meersalat und saurer Buttermilch besteht.

7. Verwendung von Mangan zum Verbessern der Lebensfähigkeit von Milchsäurebakterien in einem Produkt, welches gelagert wird, wobei das Produkt aus der Gruppe ausgewählt ist, welche aus einer fermentierten Milch, einem Molkereiprodukt-Milchsäurebakteriengetränk und einem Milchsäurebakteriengetränk besteht, wobei Mangan so formuliert wird, dass es 10 µg/100 g bis 1000 µg/100 g in dem Produkt umfasst, welches aus der Gruppe ausgewählt ist, die aus einer fermentierten Milch, einem Molkereiprodukt-Milchsäurebakteriengetränk und einem Milchsäurebakteriengetränk besteht, und wobei die Milchsäurebakterien Milchsäurebakterien sind, welche aus der Gruppe ausgewählt sind, die aus Milchsäurebakterien der Gattung Lactobacillus und Milchsäurebakterien der Gattung Pediococcus besteht.

8. Verwendung gemäß Anspruch 7, wobei das Produkt, das gelagert wird, ein Yoghurtgetränk, das gelagert wird, oder eine fermentierte Milch, die gelagert wird, ist.

9. Verwendung von manganhaltigem Material zum Verbessern der Lebensfähigkeit von Milchsäurebakterien in einem Produkt, das gelagert wird, wobei das Produkt aus der Gruppe ausgewählt ist, welche aus einer fermentierten Milch, einem Molkereiprodukt-Milchsäurebakteriengetränk und einem Milchsäurebakteriengetränk besteht, wobei Mangan so formuliert wird, dass es 10 µg/100 g bis 1000 µg/100 g in dem Produkt umfasst, welches aus der Gruppe ausgewählt ist, die aus einer fermentierten Milch, einem Molkereiprodukt-Milchsäurebakteriengetränk und einem Milchsäurebakteriengetränk besteht, und wobei die Milchsäurebakterien Milchsäurebakterien sind, welche aus der Gruppe ausgewählt sind, die aus Milchsäurebakterien der Gattung Lactobacillus und Milchsäurebakterien der Gattung Pediococcus besteht.

10. Verwendung gemäß Anspruch 9, wobei das Produkt, das gelagert wird, ein Yoghurtgetränk, das gelagert wird, oder eine fermentierte Milch, die gelagert wird, ist.

## Revendications

1. Utilisation de manganèse pour améliorer le taux de survie lors du stockage de bactéries d'acide lactique,
dans laquelle les bactéries d'acide lactique sont des bactéries d'acide lactique choisies dans le groupe constitué des bactéries d'acide lactique du genre Lactobacillus et des bactéries d'acide lactique du genre Pediococcus, et
dans laquelle le manganèse est ajouté en une quantité permettant d'en obtenir 10 µg/100 g à 1 000 µg/100 g dans un produit final, le produit final étant choisi dans le groupe constitué d'un lait fermenté, d'une boisson de produit laitier contenant des bactéries d'acide lactique et d'une boisson contenant des bactéries d'acide lactique.

2. Utilisation d'un produit contenant du manganèse pour améliorer le taux de survie lors du stockage de bactéries d'acide lactique, dans laquelle les bactéries d'acide lactique sont des bactéries d'acide lactique choisies dans le groupe constitué des bactéries d'acide lactique du genre Lactobacillus et des bactéries d'acide lactique du genre Pediococcus, et
dans laquelle le manganèse est ajouté en une quantité permettant d'en obtenir 10 µg/100 g à 1 000 µg/100 g dans un produit final, le produit final étant choisi dans le groupe constitué d'un lait fermenté, d'une boisson de produit laitier contenant des bactéries d'acide lactique et d'une boisson contenant des bactéries d'acide lactique.

3. Utilisation selon l'une quelconque des revendications 1 à 2, laquelle utilisation est destinée à améliorer le taux de survie lors du stockage de bactéries d'acide lactique dans un yaourt à boire.

4. Utilisation selon l'une quelconque des revendications 1 à 3, dans laquelle lesdites bactéries d'acide lactique sont choisies dans le groupe constitué de Lactobacillus casei, de Lactobacillus helveticus et de Pediococcus.

5. Utilisation selon l'une quelconque des revendications 1, 3 à 4, dans laquelle en tant que manganèse l'un du sulfate de manganèse, d'une levure contenant du manganèse et de bactéries d'acide lactique contenant du manganèse est utilisé.

6. Utilisation selon l'une quelconque des revendications 2 à 4, dans laquelle ledit produit contenant du manganèse est choisi dans le groupe constitué de feuilles de thé, du gingembre, du nori vert et du babeurre acide.

7. Utilisation de manganèse pour améliorer la viabilité des bactéries d'acide lactique dans un produit soumis à un stockage, le produit étant choisi dans le groupe constitué d'un lait fermenté, d'une boisson de produit laitier contenant des bactéries d'acide lactique et d'une boisson contenant des bactéries d'acide lactique, dans laquelle le manganèse est contenu en une quantité de 10 µg/100 g à 1 000 µg/100 g dans le produit choisi dans le groupe constitué d'un lait fermenté, d'une boisson de produit laitier contenant des bactéries d'acide lactique et d'une boisson contenant des bactéries d'acide lactique, et dans laquelle les bactéries d'acide lactique sont des bactéries d'acide lactique choisies dans le groupe constitué des bactéries d'acide lactique du genre Lactobacillus et des bactéries d'acide lactique du genre Pediococcus.

8. Utilisation selon la revendication 7, dans laquelle le produit soumis à un stockage est un yaourt à boire soumis à un stockage ou un lait fermenté soumis à un stockage.

9. Utilisation d'un produit contenant du manganèse pour améliorer la viabilité des bactéries d'acide lactique dans un produit soumis à un stockage, le produit étant choisi dans le groupe constitué d'un lait fermenté, d'une boisson de produit laitier contenant des bactéries d'acide lactique et d'une boisson contenant des bactéries d'acide lactique, dans laquelle le manganèse est contenu en une quantité de 10 µg/100 g à 1 000 µg/100 g dans le produit choisi dans le groupe constitué d'un lait fermenté, d'une boisson de produit laitier contenant des bactéries d'acide lactique et d'une boisson contenant des bactéries d'acide lactique, et
dans laquelle les bactéries d'acide lactique sont des bactéries d'acide lactique choisies dans le groupe constitué des bactéries d'acide lactique du genre Lactobacillus et des bactéries d'acide lactique du genre Pediococcus.

10. Utilisation selon la revendication 9, dans laquelle le produit soumis à un stockage est un yaourt à boire soumis à un stockage ou un lait fermenté soumis à un stockage.
